# EUROPÄISCHE PATENTSCHRIFT

(11) **EP 2 569 020 B1**
(45) Veröffentlichungstag und Bekanntmachung des Hinweises auf die Patenterteilung: **01.04.2020**
(21) Anmeldenummer: 11719756.6
(22) Anmeldetag: 08.04.2011
(51) Int. Cl.: A61L 2/20, B29C 49/42

(54) **VERFAHREN UND VORRICHTUNG ZUM STERILISIEREN SOWIE VORRICHTUNG ZUR BLASFORMUNG VON BEHÄLTERN**
METHOD AND DEVICE FOR STERILIZING AND DEVICE FOR BLOW-MOLDING CONTAINERS
PROCÉDÉ ET DISPOSITIF DE STÉRILISATION ET DISPOSITIF DESTINÉ AU MOULAGE PAR SOUFFLAGE DE RÉCIPIENTS

(30) Priorität: 11.05.2010 DE 102010020996
(43) Veröffentlichungstag der Anmeldung: 20.03.2013
(73) Patentinhaber: KHS Corpoplast GmbH, 22145 Hamburg (DE); KHS GmbH, 44143 Dortmund (DE)
(72) Erfinder: HEROLD, Thomas, 22926 Ahrensburg (DE); RIEGER, Harald, 22303 Hamburg (DE); KLATT, Dieter, 22147 Hamburg (DE); HAESENDONCKX, Frank, 22395 Hamburg (DE); Linke, Michael, 22159 Hamburg (DE)
(74) Vertreter: Hausfeld, Norbert
(86) Internationale Anmeldenummer: PCT/DE2011/000396
(87) Internationale Veröffentlichungsnummer: WO 2011/141011

(56) Entgegenhaltungen:
- EP-A1- 2 295 324
- WO-A1-2007/140883
- WO-A1-2009/052800
- WO-A1-2010/052068
- US-A- 3 712 784
- US-A1- 2003 165 400

## Beschreibung

Die Erfindung betrifft ein Verfahren zum Sterilisieren von Vorformlingen aus einem thermoplastischen Material, die zur Herstellung von blasgeformten Behältern vorgesehen sind sowie bei dem ein Sterilisationsmittel in einen Innenraum des Vorformlings eingeleitet wird.

Darüber hinaus betrifft die Erfindung eine Vorrichtung zum Blasformen von mindestens bereichsweise sterilen Behältern durch Verformung eines Vorformlings, die eine Zuführeinrichtung zur Beaufschlagung eines Innenraumes des Vorformlings mit einem Sterilisationsmittel aufweist.

Schließlich betrifft die Erfindung eine Vorrichtung zum Sterilisieren von Vorformlingen aus einem thermoplastischen Material, die zur Herstellung von blasgeformten Behältern vorgesehen sind. Ebenfalls ist von der Erfindung eine Vorrichtung zur Herstellung von mindestens bereichsweise sterilen Behältern aus einem thermoplastischen Material umfaßt.

Die Erfindung betrifft auch eine Vorrichtung zur Blasformung von Behältern, die mindestens eine auf einer Tragstruktur angeordnete Blasstation zur Umformung von thermoplastischen Vorformlingen in die Behälter aufweist.

WO 2010/052068 offenbart eine Vorrichtung zur Vorbehandlung von Vorformlingen aus thermoplastischem Kunststoff, bevor diese mittels eines Blasverfahrens zu Behältern umgeformt und mit Flüssigkeit befüllt werden. Bei dem Verfahren werden die Vorformlinge für das nachfolgende Blasverfahren temperiert und sterilisiert. Die Temperierung bzw. Erwärmung der Vorformlinge erfolgt mittels einer Bestrahlung mit Mikrowellen. Die Sterilisation der Vorformlinge erfolgt während der Vorbehandlung und mittels Einbringen eines flüssigen und/oder gasförmigen Sterilisationsmediums in die Vorformlinge.

Eine Herstellung von sterilen blasgeformten Behältern erfolgt typischerweise derart, daß diese Behälter nach ihrer Blasformung und vor einer Befüllung unter Verwendung von Wasserstoffperoxid oder anderen Chemikalien sterilisiert werden. Ebenfalls ist es bereits bekannt, die bei der Blasformung der Behälter als Ausgangsprodukt verwendeten Vorformlinge zu sterilisieren, insbesondere den Bereich der inneren Oberfläche dieser Vorformlinge.

Bei einer Behälterformung durch Blasdruckeinwirkung werden Vorformlinge aus einem thermoplastischen Material, beispielsweise Vorformlinge aus PET (Polyethylenterephtalat), innerhalb einer Blasmaschine unterschiedlichen Bearbeitungsstationen zugeführt. Typischerweise weist eine derartige Blasmaschine eine Heizeinrichtung sowie eine Blaseinrichtung auf, in deren Bereich der zuvor temperierte Vorformling durch biaxiale Orientierung zu einem Behälter expandiert wird. Die Expansion erfolgt mit Hilfe von Druckluft, die in den zu expandierenden Vorformling eingeleitet wird. Der verfahrenstechnische Ablauf bei einer derartigen Expansion des Vorformlings wird in der DE-OS 43 40 291 erläutert.

Der grundsätzliche Aufbau einer Blasstation zur Behälterformung wird in der DE-OS 42 12 583 beschrieben. Möglichkeiten zur Temperierung der Vorformlinge werden in der DE - OS 23 52 926 erläutert.

Innerhalb der Vorrichtung zur Blasformung können die Vorformlinge sowie die geblasenen Behälter mit Hilfe unterschiedlicher Handhabungseinrichtungen transportiert werden. Bewährt hat sich insbesondere die Verwendung von Transportdornen, auf die die Vorformlinge aufgesteckt werden. Die Vorformlinge können aber auch mit anderen Trageinrichtungen gehandhabt werden. Die Verwendung von Greifzangen zur Handhabung von Vorformlingen und die Verwendung von Spreizdornen, die zur Halterung in einen Mündungsbereich des Vorformlings einführbar sind, gehören ebenfalls zu den verfügbaren Konstruktionen.

Eine Handhabung von Behältern unter Verwendung von Übergaberädern wird beispielsweise in der DE-OS 199 06 438 bei einer Anordnung des Übergaberades zwischen einem Blasrad und einer Ausgabestrecke beschrieben.

Die bereits erläuterte Handhabung der Vorformlinge erfolgt zum einen bei den sogenannten Zweistufenverfahren, bei denen die Vorformlinge zunächst in einem Spritzgußverfahren hergestellt, anschließend zwischengelagert und erst später hinsichtlich ihrer Temperatur konditioniert und zu einem Behälter aufgeblasen werden. Zum anderen erfolgt eine Anwendung bei den sogenannten Einstufenverfahren, bei denen die Vorformlinge unmittelbar nach ihrer spritzgußtechnischen Herstellung und einer ausreichenden Verfestigung geeignet temperiert und anschließend aufgeblasen werden.

Im Hinblick auf die verwendeten Blasstationen sind unterschiedliche Ausführungsformen bekannt. Bei Blasstationen, die auf rotierenden Transporträdern angeordnet sind, ist eine buchartige Aufklappbarkeit der Formträger häufig anzutreffen. Es ist aber auch möglich, relativ zueinander verschiebliche oder andersartig geführte Formträger einzusetzen. Bei ortsfesten Blasstationen, die insbesondere dafür geeignet sind, mehrere Kavitäten zur Behälterformung aufzunehmen, werden typischerweise parallel zueinander angeordnete Platten als Formträger verwendet.

Hinsichtlich der Sterilisierung von Vorformlingen sind aus dem Stand der Technik bereits unterschiedliche Verfahren und Vorrichtungen bekannt, die jedoch alle verfahrensspezifische Nachteile aufweisen, die einer zuverlässigen Sterilisierung der Vorformlinge bei gleichzeitig hohen Durchsatzraten entgegenstehen.

In der EP-A 1 086 019 wird beispielsweise die Sterilisierung von heißen Vorformlingen mit einem heißen gasförmigen Sterilisationsmittel beschrieben. Es werden hintereinander angeordnete separate Behandlungsstationen verwendet, nämlich ein erstes Heizmodul, ein Sterilisiermodul sowie ein zweites Heizmodul. Nachteilig ist hierbei das Temperaturverhalten des Vorformlings während des Sterilisiervorganges sowie das unkontrollierte Austreten des Sterilisationsmittels aus dem Vorformling innerhalb der Heizung.

In der EP-A 1 896 245 wird ein Verfahren beschrieben, bei der vor der Heizung ein gasförmiges Sterilisationsmittel in einen kalten Vorformling eingeleitet wird und hier kondensiert. Problematisch ist hier die Sicherstellung einer vollständigen Kondensatbildung auf der gesamten Innenfläche des Vorformlings, da das einströmende heiße Sterilisationsmittel die Innenwandtemperatur des Vorformlings erhöht. Darüber hinaus tritt auch hier das Sterilisationsmittel nach seiner Verdampfung im Bereich der Heizung unkontrolliert innerhalb der Heizung aus dem Vorformling aus.

In der EP-A 2 138 298 wird eine Vorrichtung beschrieben, bei der vorsorglich sowohl vor dem verwendeten Blasmodul als auch hinter dem verwendeten Blasmodul Sterilisiereinrichtungen angeordnet sind. Hieraus resultiert ein sehr großer maschinenbaulicher Aufwand.

In der WO 2010/020530 A1 wird die Anordnung einer Sterilisiereinrichtung zwischen einer Heizung und dem Blasmodul beschrieben. Bei diesem Verfahren ist die Eintragsmenge von Sterilisationsmittel in den Bereich des Blasmoduls nur schwer vorhersehbar. Darüber hinaus ist die Austrittsmenge an Sterilisationsmittel in die Umgebung nicht kontrollierbar und eine entsprechende Kontamination nicht ausgeschlossen.

Aufgabe der vorliegenden Erfindung ist es, ein Verfahren der einleitend genannten Art derart zu verbessern, daß in einfacher Weise eine zuverlässige Sterilisation durchgeführt werden kann.

Diese Aufgabe wird erfindungsgemäß dadurch gelöst, daß in einem Mündungsabschnitt des Vorformlings mindestens während eines Teiles der Zeitdauer der Sterilisierung ein Zuführelement für das Sterilisationsmittel positioniert wird.

Weitere Aufgabe der vorliegenden Erfindung ist es, eine Vorrichtung der einleitend genannten Art derart zu konstruieren, daß eine effektive Sterilisation mit geringem Aufwand durchführbar ist.

Diese Aufgabe wird erfindungsgemäß dadurch gelöst, daß die Zuführeinrichtung einen Applikator für das Sterilisationsmittel aufweist, der in einem Mündungsbereich des Vorformlings positionierbar ist.

Schließlich besteht eine Aufgabe der vorliegenden Erfindung darin, eine Vorrichtung zur Blasformung von Behältern der einleitend genannten Art derart zu konstruieren, daß die Herstellung von sterilen Behältern unterstützt wird.

Diese Aufgabe wird erfindungsgemäß durch den Gegenstand der unabhängigen Ansprüche gelöst.

Die erfindungsgemäße Konstruktion unterstützt insbesondere auch einen Transport von sterilen Vorformlingen und/oder Behältern innerhalb der Blasmaschine.

Eine Abdichtung des Innenraumes des Vorformlings gegenüber einer Umgebung bei der Durchführung der Sterilisation wird dadurch unterstützt, daß das Zuführelement in den Mündungsabschnitt eingeführt wird.

Eine sichere Handhabung wird dadurch unterstützt, daß das Zuführelement relativ zum Mündungsabschnitt fixiert wird.

Insbesondere ist daran gedacht, daß das Zuführelement relativ zum Vorformling verspannt wird.

Ein unkontrolliertes Entweichen von Sterilisationsmittel kann dadurch verhindert werden, daß das Sterilisationsmittel durch mindestens einen Zuführkanal eines Applikators des Zuführelementes in den Vorformling eingeführt und durch mindestens einen Ableitkanal des Applikators aus dem Vorformling abgeleitet wird.

Insbesondere ist daran gedacht, daß das Sterilisationsmittel aus dem Vorformling heraus einer Ableiteinrichtung zugeführt wird.

Eine erhöhte Sterilisierwirkung kann dadurch erreicht werden, daß die Sterilisierung mit einem Sterilisationsmittel durchgeführt wird, das eine Temperatur oberhalb von 100°C aufweist.

Darüber hinaus trägt es zur Optimierung des Sterilisationsvorganges bei, daß die Sterilisierung bei einer Temperatur des Vorformlings durchgeführt wird, die mindestens bereichsweise oberhalb von einer Glastemperatur des Materials des Vorformlings liegt. Bei einer Verarbeitung von PET ist insbesondere daran gedacht, eine Temperatur oberhalb von 80°C vorzusehen.

In den Zeichnungen sind Ausführungsbeispiele der Erfindung schematisch dargestellt. Es zeigen:
- Fig. 1: Eine perspektivische Darstellung einer Blasstation zur Herstellung von Behältern aus Vorformlingen,
- Fig. 2: einen Längsschnitt durch eine Blasform, in der ein Vorformling gereckt und expandiert wird,
- Fig. 3: eine Skizze zur Veranschaulichung eines grundsätzlichen Aufbaus einer Vorrichtung zur Blasformung von Behältern,
- Fig. 4: eine modifizierte Heizstrecke mit vergrößerter Heizkapazität,
- Fig. 5: eine schematische Darstellung eines Heizmoduls einer Blasmaschine, bei der im Bereich des Heizmoduls eine Sterilisiereinrichtung angeordnet ist,
- Fig. 6: eine gegenüber Fig. 5 abgewandelte Ausführungsform,
- Fig. 7: einen Längsschnitt durch einen Applikator für das Sterilisationsmittel, der in einen Mündungsbereich des Vorformlings eingeführt ist und
- Fig. 8: eine gegenüber Fig. 7 abgewandelte, erfindungsgemäße Ausführungsform, bei der der Applikator sowohl eine gesteuerte Zuführung als auch eine gesteuerte Ableitung des Sterilisationsmittels ermöglicht.

Vor einer Erläuterung des Detailaufbaus der Vorrichtung zum Sterilisieren der Vorformlinge (1) durch Anwendung eines Sterilisationsmittels sowie vor einer Erläuterung eines konkreten Einbaues einer entsprechenden Vorrichtung in eine Blasmaschine soll nachfolgend zunächst der grundsätzliche Aufbau einer Blasmaschine beschrieben werden.

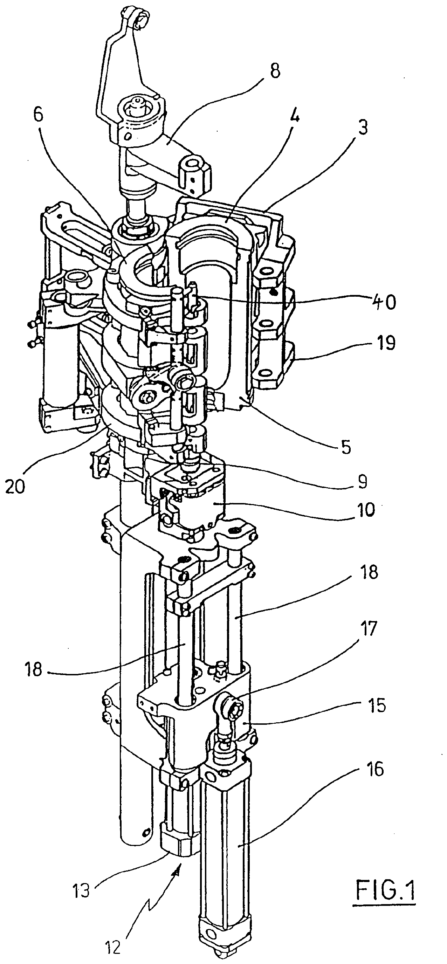

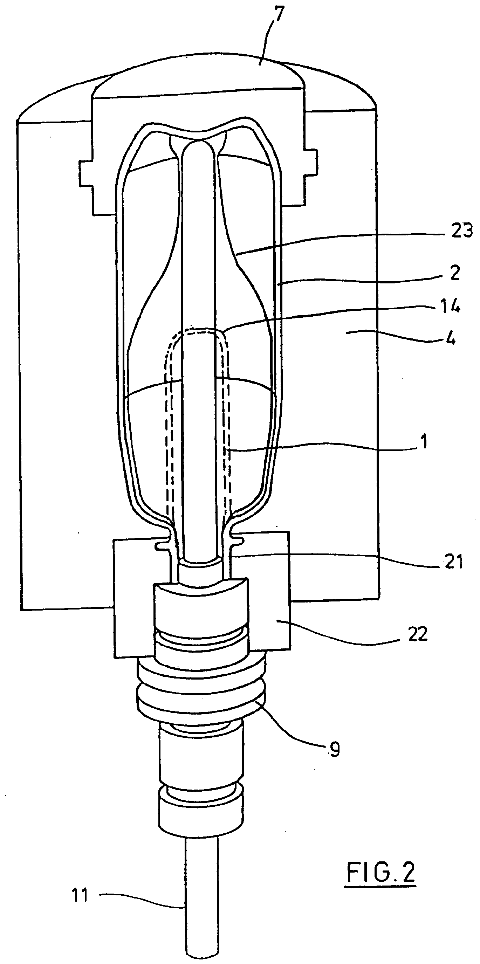

Der prinzipielle Aufbau einer Vorrichtung zur Umformung von Vorformlingen (1) in Behälter (2) ist in Fig. 1 und in Fig. 2 dargestellt.

Die Vorrichtung zur Formung des Behälters (2) besteht im Wesentlichen aus einer Blasstation (3), die mit einer Blasform (4) versehen ist, in die ein Vorformling (1) einsetzbar ist. Der Vorformling (1) kann ein spritzgegossenes Teil aus Polyethylenterephthalat sein. Zur Ermöglichung eines Einsetzens des Vorformlings (1) in die Blasform (4) und zur Ermöglichung eines Herausnehmens des fertigen Behälters (2) besteht die Blasform (4) aus Formhälften (5, 6) und einem Bodenteil (7), das von einer Hubvorrichtung (8) positionierbar ist. Der Vorformling (1) kann im Bereich der Blasstation (3) von einem Transportdorn (9) gehalten sein, der gemeinsam mit dem Vorformling (1) eine Mehrzahl von Behandlungsstationen innerhalb der Vorrichtung durchläuft. Es ist aber auch möglich, den Vorformling (1) beispielsweise über Zangen oder andere Handhabungsmittel direkt in die Blasform (4) einzusetzen.

Zur Ermöglichung einer Druckluftzuleitung ist unterhalb des Transportdornes (9) ein Anschlußkolben (10) angeordnet, der dem Vorformling (1) Druckluft zuführt und gleichzeitig eine Abdichtung relativ zum Transportdorn (9) vornimmt. Bei einer abgewandelten Konstruktion ist es grundsätzlich aber auch denkbar, feste Druckluftzuleitungen zu verwenden.

Eine Reckung des Vorformlings (1) erfolgt mit Hilfe einer Reckstange (11), die von einem Zylinder (12) positioniert wird. Grundsätzlich ist es aber auch denkbar, eine mechanische Positionierung der Reckstange (11) über Kurvensegmente durchzuführen, die von Abgriffrollen beaufschlagt sind. Die Verwendung von Kurvensegmenten ist insbesondere dann zweckmäßig, wenn eine Mehrzahl von Blasstationen (3) auf einem rotierenden Blasrad angeordnet sind. Eine Verwendung von Zylindern (12) ist zweckmäßig, wenn ortsfest angeordnete Blasstationen (3) vorgesehen sind.

Bei der in Fig. 1 dargestellten Ausführungsform ist das Recksystem derart ausgebildet, daß eine Tandem-Anordnung von zwei Zylindern (12) bereitgestellt ist. Von einem Primärzylinder (13) wird die Reckstange (11) zunächst vor Beginn des eigentlichen Reckvorganges bis in den Bereich eines Bodens (14) des Vorformlings (1) gefahren. Während des eigentlichen Reckvorganges wird der Primärzylinder (13) mit ausgefahrener Reckstange gemeinsam mit einem den Primärzylinder (13) tragenden Schlitten (15) von einem Sekundärzylinder (16) oder über eine Kurvensteuerung positioniert. Insbesondere ist daran gedacht, den Sekundärzylinder (16) derart kurvengesteuert einzusetzen, daß von einer Führungsrolle (17), die während der Durchführung des Reckvorganges an einer Kurvenbahn entlang gleitet, eine aktuelle Reckposition vorgegeben wird. Die Führungsrolle (17) wird vom Sekundärzylinder (16) gegen die Führungsbahn gedrückt. Der Schlitten (15) gleitet entlang von zwei Führungselementen (18).

Nach einem Schließen der im Bereich von Trägern (19, 20) angeordneten Formhälften (5, 6) erfolgt eine Verriegelung der Träger (19, 20) relativ zueinander mit Hilfe einer Verriegelungseinrichtung (40).

Zur Anpassung an unterschiedliche Formen eines Mündungsabschnittes (21) des Vorformlings (1) ist gemäß Fig. 2 die Verwendung separater Gewindeeinsätze (22) im Bereich der Blasform (4) vorgesehen.

Fig. 2 zeigt zusätzlich zum geblasenen Behälter (2) auch gestrichelt eingezeichnet den Vorformling (1) und schematisch eine sich entwickelnde Behälterblase (23).

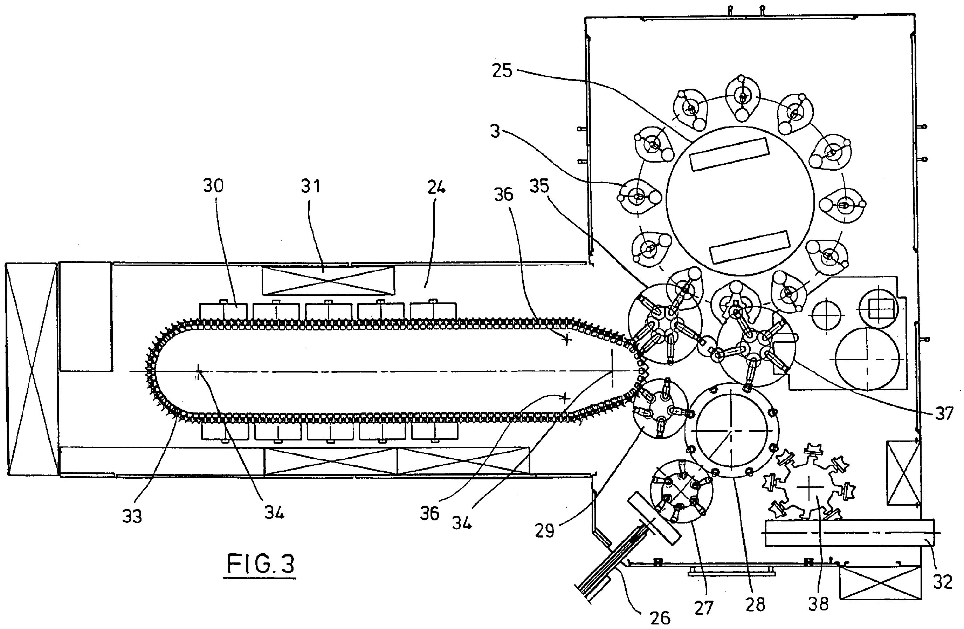

Fig. 3 zeigt den grundsätzlichen Aufbau einer Blasmaschine, die mit einer Heizstrecke (24) sowie einem rotierenden Blasrad (25) versehen ist. Ausgehend von einer Vorformlingseingabe (26) werden die Vorformlinge (1) von Übergaberädern (27, 28, 29) in den Bereich der Heizstrecke (24) transportiert. Entlang der Heizstrecke (24) sind Heizstrahler (30) sowie Gebläse (31) angeordnet, um die Vorformlinge (1) zu temperieren. Nach einer ausreichenden Temperierung der Vorformlinge (1) werden diese an das Blasrad (25) übergeben, in dessen Bereich die Blasstationen (3) angeordnet sind. Die fertig geblasenen Behälter (2) werden von weiteren Übergaberädern einer Ausgabestrecke (32) zugeführt.

Um einen Vorformling (1) derart in einen Behälter (2) umformen zu können, daß der Behälter (2) Materialeigenschaften aufweist, die eine lange Verwendungsfähigkeit von innerhalb des Behälters (2) abgefüllten Lebensmitteln, insbesondere von Getränken, gewährleisten, müssen spezielle Verfahrensschritte bei der Beheizung und Orientierung der Vorformlinge (1) eingehalten werden. Darüber hinaus können vorteilhafte Wirkungen durch Einhaltung spezieller Dimensionierungsvorschriften erzielt werden.

Als thermoplastisches Material können unterschiedliche Kunststoffe verwendet werden. Einsatzfähig sind beispielsweise PET, PEN oder PP.

Die Expansion des Vorformlings (1) während des Orientierungsvorganges erfolgt durch Druckluftzuführung. Die Druckluftzuführung ist in eine Vorblasphase, in der Gas, zum Beispiel Preßluft, mit einem niedrigen Druckniveau zugeführt wird und in eine sich anschließende Hauptblasphase unterteilt, in der Gas mit einem höheren Druckniveau zugeführt wird. Während der Vorblasphase wird typischerweise Druckluft mit einem Druck im Intervall von 10 bar bis 25 bar verwendet und während der Hauptblasphase wird Druckluft mit einem Druck im Intervall von 25 bar bis 40 bar zugeführt.

Aus Fig. 3 ist ebenfalls erkennbar, daß bei der dargestellten Ausführungsform die Heizstrecke (24) aus einer Vielzahl umlaufender Transportelemente (33) ausgebildet ist, die kettenartig aneinandergereiht und entlang von Umlenkrädern (34) geführt sind. Insbesondere ist daran gedacht, durch die kettenartige Anordnung eine im Wesentlichen rechteckförmige Grundkontur aufzuspannen. Bei der dargestellten Ausführungsform werden im Bereich der dem Übergaberad (29) und einem Eingaberad (35) zugewandten Ausdehnung der Heizstrecke (24) ein einzelnes relativ groß dimensioniertes Umlenkrad (34) und im Bereich von benachbarten Umlenkungen zwei vergleichsweise kleiner dimensionierte Umlenkräder (36) verwendet. Grundsätzlich sind aber auch beliebige andere Führungen denkbar.

Zur Ermöglichung einer möglichst dichten Anordnung des Übergaberades (29) und des Eingaberades (35) relativ zueinander erweist sich die dargestellte Anordnung als besonders zweckmäßig, da im Bereich der entsprechenden Ausdehnung der Heizstrecke (24) drei Umlenkräder (34, 36) positioniert sind, und zwar jeweils die kleineren Umlenkräder (36) im Bereich der Überleitung zu den linearen Verläufen der Heizstrecke (24) und das größere Umlenkrad (34) im unmittelbaren Übergabebereich zum Übergaberad (29) und zum Eingaberad (35). Alternativ zur Verwendung von kettenartigen Transportelementen (33) ist es beispielsweise auch möglich, ein rotierendes Heizrad zu verwenden.

Nach einem fertigen Blasen der Behälter (2) werden diese von einem Entnahmerad (37) aus dem Bereich der Blasstationen (3) herausgeführt und über das Übergaberad (28) und ein Ausgaberad (38) zur Ausgabestrecke (32) transportiert.

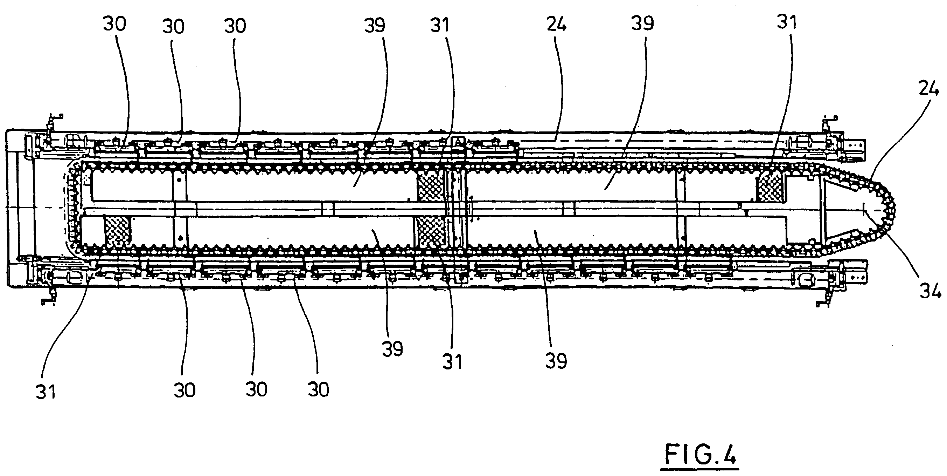

In der in Fig. 4 dargestellten modifizierten Heizstrecke (24) können durch die größere Anzahl von Heizstrahlern (30) eine größere Menge von Vorformlingen (1) je Zeiteinheit temperiert werden. Die Gebläse (31) leiten hier Kühlluft in den Bereich von Kühlluftkanälen (39) ein, die den zugeordneten Heizstrahlern (30) jeweils gegenüberliegen und über Ausströmöffnungen die Kühlluft abgeben. Durch die Anordnung der Ausströmrichtungen wird eine Strömungsrichtung für die Kühlluft im Wesentlichen quer zu einer Transportrichtung der Vorformlinge (1) realisiert. Die Kühlluftkanäle (39) können im Bereich von den Heizstrahlern (30) gegenüberliegenden Oberflächen Reflektoren für die Heizstrahlung bereitstellen, ebenfalls ist es möglich, über die abgegebene Kühlluft auch eine Kühlung der Heizstrahler (30) zu realisieren.

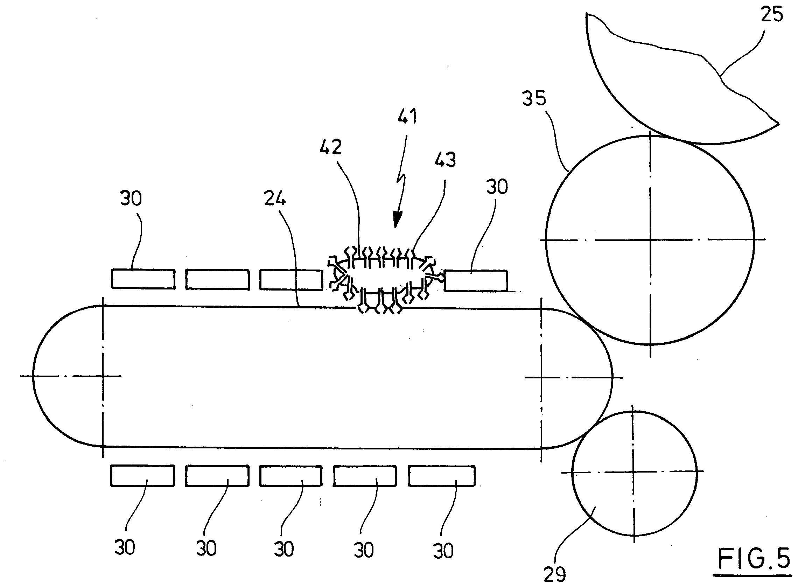

Fig. 5 zeigt schematisch und stark vereinfacht eine Anordnung ähnlich zur Darstellung in Fig. 3 mit zusätzlicher Anordnung einer Sterilisiereinrichtung (41) im Bereich der Heizstrecke (24). Gemäß dem in Fig. 5 dargestellten Ausführungsbeispiel ist im Bereich der Sterilisiereinrichtung (41) eine umlaufend angetriebene Transporteinrichtung (42) für Halteelemente (43) angeordnet, die eine Fixierung und/oder Positionierung der Vorformlinge (1) mindestens während eines Teiles der Zeitdauer des Sterilisationsvorganges ermöglichen.

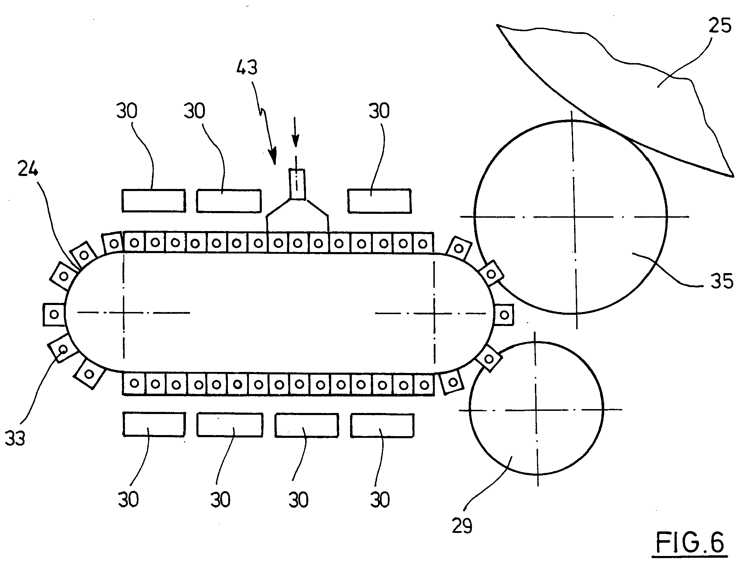

Fig. 6 zeigt eine abgewandelte Ausführungsform, bei der im Bereich der Sterilisiereinrichtung (41) keine zusätzlichen Halteelemente (42) verwendet werden. Zusätzlich eingezeichnet sind die Transportelemente (33) der Heizstrecke (24).

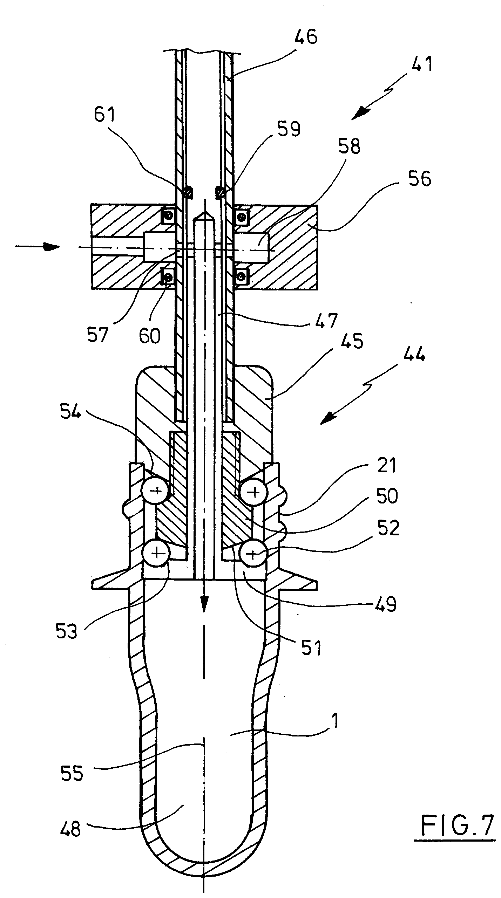

Fig. 7 zeigt einen Bereich der Sterilisiereinrichtung (41), die mit einem Applikator (44) in den Mündungsabschnitt (21) des Vorformlings (1) eingeführt ist. Der Applikator (44) weist ein Basiselement (45) auf, das von einem Positionierelement (46) getragen ist. Beim dargestellten Ausführungsbeispiel ist das Positionierelement (46) rohrartig ausgebildet und durch das Positionierelement (46) erstreckt sich ein Stellelement (47) hindurch. Das Stellelement (47) mündet im Bereich seiner einem Innenraum (48) des Vorformlings (1) zugewandten Ausdehnung in ein Spannelement (49) ein.

Zwischen dem Spannelement (49) und dem Basiselement (45) ist ein Keilelement (50) angeordnet, das mindestens eine Keilfläche (51) zur Beaufschlagung mindestens eines Klemmelementes (52) aufweist. Das Klemmelement (52) ist gerundet ausgebildet. Vorzugsweise liegt eine kugelartige Ausführung des Klemmelementes (52) vor. Als Material für das Klemmelement (52) ist beispielsweise an die Verwendung einer Keramik, von PEEK oder einem mit Keramik beschichteten Metall gedacht.

Im Bereich seiner dem Keilelement (50) zugewandten Ausdehnung weist das Spannelement (49) mindestens eine Keilfläche (53) auf. Ebenfalls ist das Basiselement (45) im Bereich seiner dem Keilelement (50) zugewandten Ausdehnung mit mindestens einer Keilfläche (54) versehen.

Beim dargestellten Ausführungsbeispiel sind somit in Richtung einer Längsachse (55) des Positionierelementes (46) hintereinander und relativ zueinander mit einem Abstand mindestens zwei Klemmelemente (52) positioniert. Dies ist aber optional und nicht zwingend notwendig. Ebenfalls sind in einer Umfangsrichtung des Keilelementes (50) relativ zueinander beabstandet mindestens zwei Klemmelemente (52) angeordnet. Bevorzugt werden mindestens drei in der Umfangsrichtung relativ zueinander äquidistant beabstandete Klemmelemente (52) verwendet.

Für ein Einführen des Applikators (44) in den Mündungsabschnitt (21) bzw. für ein Entfernen des Applikators (44) aus dem Bereich des Mündungsabschnittes (21) wird gegenüber der Positionierung in Fig. 7 der Abstand zwischen dem Spannelement (49) und dem Basiselement (45) durch eine Positionierung des Stellelementes (47) vergrößert. Die Klemmelemente (52) können hierdurch in Richtung auf das Stellelement (47) weiter in jeweils durch Keilflächen (51, 52) bzw. Keilflächen (51, 54) begrenzte Aufnahmetaschen hineinwandern und geben den Vorformling (1) relativ zum Applikator (44) frei. In umgekehrter Weise werden die Klemmelemente (52) bei einer Abstandsverringerung zwischen dem Spannelement (49) und dem Basiselement (45) entlang der Keilflächen (51, 53, 54) in Richtung auf den Mündungsabschnitt (21) des Vorformlings (1) positioniert und führen hierdurch einen Klemmvorgang durch.

Nach einer Positionierung des Applikators (44) im Bereich des Mündungsabschnittes (41) wird unter Verwendung einer Zuführeinrichtung (56) Sterilisationsmittel in den Bereich des Applikators (44) geleitet. Beim dargestellten Ausführungsbeispiel weist das rohrartige Positionierelement (46) mindestens eine außenseitige Ausnehmung (57) auf, die in einen Zuführraum (58) der Zuführeinrichtung (56) einmündet. Der Zuführraum (58) ist durch Dichtungen (59, 60) gegenüber einer Umgebung abgedichtet. Die Dichtungen (59, 60) können beispielsweise als O-Ringe ausgebildet sein, die das Positionierelement (46) außenseitig umgeben.

Zur Abdichtung des Stellelementes (47) relativ zum Positionierelement (46) wird eine Dichtung (61) verwendet. Auch diese Dichtung (41) kann als ein O-Ring ausgebildet sein, der in eine außenseitige nutförmige Vertiefung des Stellelementes (47) eingesetzt ist.

Zur Durchführung eines Sterilisationsvorganges wird durch die Zuführeinrichtung (56) und das Positionierelement (46) hindurch das Sterilisationsmittel in den Vorformling (1) eingeleitet. Beim dargestellten Ausführungsbeispiel ist auch das Stellelement (47) zumindest bereichsweise hohl ausgebildet, so daß die Zuführung des Sterilisationsmittels durch den hohlen Bereich des Stellelementes (47) hindurch erfolgt. Hierdurch kann eine zentrale Einleitung in den Vorformling (1) in Richtung der Längsachse (55) erfolgen. Eine Ableitung des Sterilisationsmittels aus dem Innenraum (48) heraus kann beispielsweise durch Kanäle oder Nuten im Bereich des Applikators (44) erfolgen. Im einfachsten Fall erfolgt eine Ableitung durch geeignete Nuten an den Klemmelementen (52) vorbei in Richtung auf eine Umgebung.

Das Sterilisationsmittel wird vorzugsweise in einem gasförmigen Zustand in den Innenraum (48) eingeleitet. Insbesondere ist an eine Temperatur des Sterilisationsmittels von oberhalb 100°C gedacht. Vorzugsweise weist der Vorformling (1) bei der Durchführung des Sterilisationsvorganges im Bereich seiner zu sterilisierenden inneren Oberfläche eine Temperatur von oberhalb 80°C auf. Hinsichtlich des Sterilisationsmittels ist insbesondere an die Verwendung von Wasserstoffperoxid gedacht.

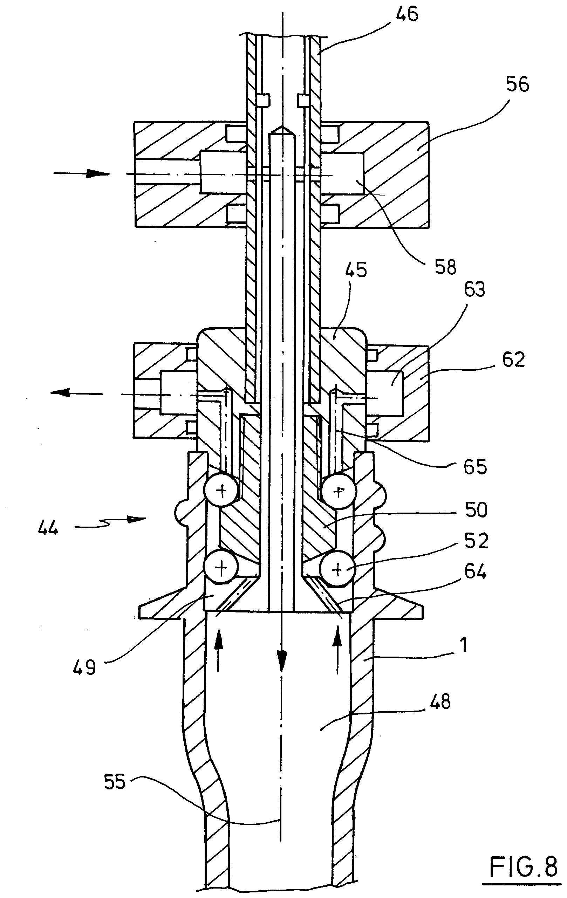

Fig. 8 zeigt eine gegenüber der Ausführungsform in Fig. 7 abgewandelte Ausführungsform. Zusätzlich zur Zuführeinrichtung (56) wird hier eine Ableiteinrichtung (62) für wieder aus dem Innenraum (48) ausströmendes Sterilisationsmittel verwendet. Die Ableiteinrichtung (62) ist im Wesentlichen ähnlich zur Zuführeinrichtung (56) ausgebildet. Zur Verbindung eines Ableitraumes (63) mit dem Innenraum (48) des Vorformlings (1) weisen das Spannelement (49) und das Basiselement (45) jeweils mindestens einen Strömungskanal (64, 65) auf. Beim dargestellten Ausführungsbeispiel erstreckt sich die Ableiteinrichtung (62) mindestens bereichsweise außenseitlich zum Basiselement (45). Zur Vereinfachung der Konstruktion kann auch hier vorgesehen sein, daß am Keilelement (50) vorbei eine Ableitung des Sterilisationsmittels über die für die Klemmelemente (52) vorgesehenen Taschen und über Abstandsbereiche zwischen dem Keilelement (50) und dem Mündungsabschnitt (21) erfolgt.

Gemäß der in Fig. 8 vorgesehenen Strömungsführung erfolgt die Zuleitung des Sterilisationsmittels zentral entlang der Längsachse (55) in den Vorformling hinein. Einströmöffnungen der Kanäle (64) sind benachbart zu einem Außenumfang des Spannelementes (49) angeordnet. Die Anordnung erfolgt somit mit einem relativ geringen Abstand zur Wandung des Vorformlings (1). Hierdurch wird das abströmende Sterilisationsmittel an der Innenseite der Wandung des Vorformlings (1) vorbeigeführt.

Die Verwendung des am Positionierelement (46) befestigten Applikators (44) kann in unterschiedlichen Varianten erfolgen. Beispielsweise ist es möglich, den Applikator (44) im gesamten Bereich der Heizstrecke (24) als Transportelement (33) zu benutzen. Gemäß einer anderen Variante ist es denkbar, im Bereich der Sterilisiereinrichtung (41) die Transportelemente (33) zu entfernen und durch den Applikator (44) zu ersetzen.

Alternativ zur vorstehend beschriebenen Befestigung des Applikators (44) innerhalb des Mündungsbereiches des Vorformlings (1) ist es auch möglich, den Applikator (44) außenseitig am Mündungsbereich des Vorformlings (1) anzuordnen. Hierdurch wird eine haubenartige Gestaltung bereitgestellt.

Bei einer Kombination der Blasmaschine mit einem nachfolgenden Füller ist es möglich, die Innenräume der jeweiligen Maschinen durch eine Luftschleuse voneinander zu trennen. Hierdurch wird ein Eintrag von Feuchtigkeit durch rücklaufende Transportelemente vom Füller in die Blasmaschine vermieden. Darüber hinaus wird auch ein Eintrag von Luft, die noch Sterilisationsmittel enthält, aus der Blasmaschine in den Bereich des Füllers vermieden. Hieraus resultiert die Vermeidung einer Kontamination von in den geblasenen Behälter abzufüllenden Getränken mit dem Sterilisationsmittel.

Zusätzlich zur Sterilisierung der Vorformlinge ist es auch möglich, eine Sterilisation der äußeren Oberfläche des geblasenen Behälters und/oder des Mündungsbereiches des Behälters durchzuführen. Gemäß einer Ausführungsform der Erfindung werden die geblasenen Behälter an dem Ausgangsbereich der Blasmaschine von zangenartigen Halteelementen am Stützring gehalten. Der Mündungsbereich ist hierdurch frei zugänglich und kann sterilisiert werden. Durch die vorstehend beschriebene Außensterilisation der geblasenen Behälter wird ein Eintrag von Keimen, die auf der äußeren Oberfläche anhaften, in den Sterilraum des Füllers vermieden.

## Patentansprüche

1. Verfahren zum Sterilisieren von Vorformlingen (1) aus einem thermoplastischen Material, die zur Herstellung von blasgeformten Behältern (2) vorgesehen sind sowie bei dem ein Sterilisationsmittel in einem Innenraum (48) des Vorformlings (1) eingeleitet wird, wobei in einem Mündungsbereich (21) des Vorformlings (1) mindestens während eines Teiles der Zeitdauer der Sterilisierung ein Zuführelement für das Sterilisationsmittel positioniert wird, **dadurch gekennzeichnet, dass** das Sterilisationsmittel durch mindestens einen Zuführkanal (47) eines Applikators (44) des Zuführelementes in den Vorformling (1) eingeführt und durch mindestens einen Ableitkanal (64) des Applikators (44) oder Nuten im Bereich des Applikators (44) aus dem Vorformling (1) abgeleitet wird, wobei
das Zuführelement in den Mündungsabschnitt (21) eingeführt und relativ zum Vorformling (1) verspannt wird.

2. Verfahren nach Anspruch 1, **dadurch gekennzeichnet, dass** das Zuführelement relativ zum Mündungsabschnitt (21) fixiert wird.

3. Verfahren nach Anspruch 1, **dadurch gekennzeichnet, dass** das Sterilisationsmittel aus dem Vorformling (1) heraus einer Ableiteinrichtung zugeführt wird.

4. Verfahren nach einem der Ansprüche 1 bis 3, **dadurch gekennzeichnet, dass** die Sterilisierung mit einem Sterilisationsmittel durchgeführt wird, das eine Temperatur oberhalb von 100°C aufweist.

5. Verfahren nach einem der Ansprüche 1 bis 4, **dadurch gekennzeichnet, dass** die Sterilisierung bei der Temperatur des Vorformlings (1) durchgeführt wird, die mindestens bereichsweise oberhalb einer Glastemperatur des Materials des Vorformlings (1) liegt.

6. Vorrichtung zum Sterilisieren von Vorformlingen (1) aus einem thermoplastischen Material, die zur Herstellung von blasgeformten Behältern (2) vorgesehen sind, die eine Zuführeinrichtung zur Beaufschlagung eines Innenraumes (48) des Vorformlings (1) mit einem Sterilisationsmittel aufweist, wobei die Zuführeinrichtung (56) einen Applikator (44) für das Sterilisationsmittel aufweist, der in einem Mündungsbereich des Vorformlings (1) positionierbar ist, **dadurch gekennzeichnet, dass** der Applikator (44) mindestens einen Zuführkanal (47) für das Sterilisationsmittel sowie mindestens einen Ableitkanal (64) für das Sterilisationsmittel aufweist oder Ableitnuten im Bereich des Applikators angeordnet sind, wobei
der Applikator (44) in einen Mündungsabschnitt (21) des Vorformlings (1) einführbar ist und im Bereich des Mündungsabschnittes (21) gegenüber dem Vorformling (1) verspannbar ist.

7. Vorrichtung nach Anspruch 6, **dadurch gekennzeichnet, dass** der Applikator (44) im Bereich des Mündungsabschnittes (21) fixierbar ist.

8. Vorrichtung nach einem der Ansprüche 6 bis 7, **dadurch gekennzeichnet, dass** der Ableitkanal mit einer Ableiteinrichtung verbindbar ist.

9. Vorrichtung nach einem der Ansprüche 6 bis 8, **dadurch gekennzeichnet, dass** eine Blasmaschine zur Umformung der Vorformlinge (1) in Behälter (2) im Bereich ihrer Heizstrecke (24) mit der Sterilisiereinrichtung (41) versehen ist.

## Claims

1. A method for sterilising preforms (1) of a thermoplastic material, which are provided for producing blow-moulded containers (2), and in the case of which a sterilisation agent is introduced into an interior (48) of the preform (1), wherein a feed element for the sterilisation agent is positioned in an opening region (21) of the preform (1) at least during a part of the duration of the sterilisation, **characterised in that** the sterilisation agent is introduced into the preform (1) through at least one feed channel (47) of an applicator (44) of the feed element and is discharged from the preform (1) through at least one discharge channel (64) of the applicator (44) or grooves in the region of the applicator (44), wherein
the feed element is inserted into the opening section (21) and is clamped relative to the preform (1).

2. The method according to Claim 1, **characterised in that** the feed element is fixed relative to the opening section (21).

3. The method according to Claim 1, **characterised in that** the sterilisation agent is fed out of the preform (1) to a discharge device.

4. The method according to any one of Claims 1 to 3, **characterised in that** the sterilisation is performed with a sterilisation agent, which has a temperature of above 100°C.

5. The method according to any one of Claims 1 to 4, **characterised in that** the sterilisation is performed at the temperature of the preform (1), which at least in some regions lies above a glass temperature of the material of the preform (1).

6. A device for sterilising preforms (1) of a thermoplastic material, which are provided for producing blow-moulded containers (2), which has a feed device for applying a sterilisation agent to an interior (48) of the preform (1), wherein the feed device (56) has an applicator (44) for the sterilisation agent, which can be positioned in an opening region of the preform (1), **characterised in that** the applicator (44) has at least one feed channel (47) for the sterilisation agent as well as at least one discharge channel (64) for the sterilisation agent or discharge grooves are arranged in the region of the applicator, wherein
the applicator (44) can be inserted into an opening section (21) of the preform (1) and can be clamped in the region of the opening section (21) relative to the preform (1).

7. The device according to Claim 6, **characterised in that** the applicator (44) can be fixed in the region of the opening section (21).

8. The device according to any one of Claims 6 to 7, **characterised in that** the discharge channel can be connected to a discharge device.

9. The device according to any one of Claims 6 to 8, **characterised in that** a blowing machine for forming the preforms (1) into containers (2) is provided with the sterilisation device (41) in the region of its heating section (24).

## Revendications

1. Procédé de stérilisation de préformes (1) en un matériau thermoplastique destinées à la fabrication de récipients (2) moulés par soufflage, dans le cadre duquel un agent de stérilisation est introduit dans un espace intérieur (48) de la préforme (1), un élément d'adduction pour l'agent de stérilisation étant positionné dans une section d'embouchure (21) de la préforme (1) pendant au moins une partie de la durée de la stérilisation, **caractérisé en ce que** l'agent de stérilisation est introduit dans la préforme (1) par au moins un canal d'adduction (47) d'un applicateur (44) de l'élément d'adduction et en est évacué par au moins un canal d'évacuation (64) de l'applicateur (44) ou par des rainures au niveau de l'applicateur (44), l'élément d'adduction étant introduit dans la section d'embouchure (21) et serré par rapport à la préforme (1).

2. Procédé selon la revendication 1, **caractérisé en ce que** l'élément d'adduction est fixé par rapport à la section d'embouchure (21).

3. Procédé selon la revendication 1, **caractérisé en ce que** l'agent de stérilisation est conduit hors de la préforme (1) vers un dispositif d'évacuation.

4. Procédé selon l'une des revendications 1 à 3, **caractérisé en ce que** la stérilisation est effectuée avec un agent de stérilisation qui présente une température supérieure à 100°C.

5. Procédé selon l'une des revendications 1 à 4, **caractérisé en ce que** la stérilisation est effectuée à la température de la préforme (1) qui en partie au moins, est supérieure à la température de transition vitreuse du matériau de la préforme (1).

6. Dispositif de stérilisation de préformes (1) en un matériau thermoplastique destinées à la fabrication de récipients moulés par soufflage présentant un dispositif d'adduction d'un agent de stérilisation agissant sur un espace intérieur (48) de la préforme (1), le dispositif d'adduction (56) présentant un applicateur (44) pour l'application de l'agent de stérilisation pouvant être positionné dans une section d'embouchure de la préforme (1), **caractérisé en ce que** l'applicateur (44) présente au moins un canal d'adduction (47) pour l'agent de stérilisation ainsi qu'au moins un canal d'évacuation (64) pour l'agent de stérilisation ou des rainures d'évacuation aménagées au niveau de l'applicateur, l'applicateur (44) pouvant être introduit dans une section d'embouchure (21) de la préforme (1) et serré par rapport à la préforme (1) au niveau de la section d'embouchure (21).

7. Dispositif selon la revendication 6, **caractérisé en ce que** l'applicateur (44) peut être fixé au niveau de la section d'embouchure (21).

8. Dispositif selon l'une des revendications 6 à 7, **caractérisé en ce que** le canal d'évacuation peut être raccordé à un dispositif d'évacuation.

9. Dispositif selon l'une des revendications 6 à 8, **caractérisé en ce qu'**une machine de moulage par soufflage pour la transformation des préformes (1) en récipients (2) est munie d'un dispositif de stérilisation (41) dans sa zone de chauffage (24).
